(19) Europäisches Patentamt
European Patent Office
Office européen des brevets

(11) Publication number: 0 394 813 A2

# (12) EUROPEAN PATENT APPLICATION

(21) Application number: 90107294.2

(51) Int. Cl.$^5$: C08G 63/60

(22) Date of filing: 18.04.90

(30) Priority: 26.04.89 US 343473
19.05.89 US 354065

(43) Date of publication of application:
31.10.90 Bulletin 90/44

(84) Designated Contracting States:
AT BE CH DE ES FR GB IT LI NL SE

(71) Applicant: THE DOW CHEMICAL COMPANY
2030 Dow Center Abbott Road P.O. Box 1967
Midland Michigan 48640-1967(US)

(72) Inventor: Lee, Guo-shuh John
10 Burrell Court
Midland, Michigan 48640(US)

Inventor: Burdett, Kenneth A.
2800 Diana
Midland, Michigan 48640(US)
Inventor: Brewbaker, James L.
304 Nakoma
Midland, Michigan 48640(US)
Inventor: Marshall, William B.
3521 W. Shamrock
Midland, Michigan 48640(US)

(74) Representative: Sternagel, Hans-Günther, Dr. et al
Patentanwälte Dr. Michael Hann Dr. H.-G. Sternagel Sander Aue 30
D-5060 Bergisch Gladbach 2(DE)

## (54) Melt processable thermotropic aromatic copolyesters and process for preparing same.

(57) Disclosed is a copolymer capable of forming an optically anisotropic melt comprising recurring structural units of Formulas I, II III; and IV:

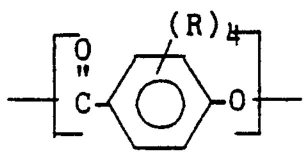
I,

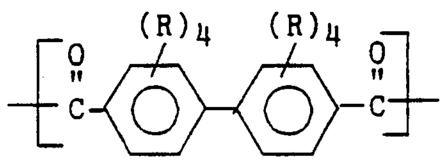
II,

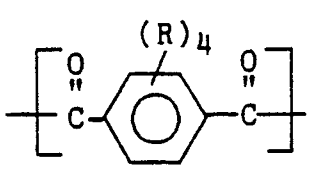
III,

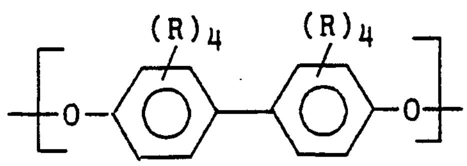
IV

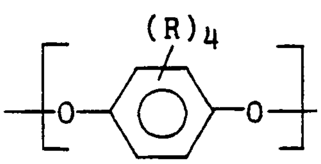
V,

EP 0 394 813 A2

wherein each R is independently a chemically inert substituent. The present invention also relates to a process for preparing the copolymer and to a process for preparing dihydroxy polyphenyls which can be used as starting material to prepare the copolymer. The copolymers of the present invention usually have a low melting point below 350° C and can be readily melt extruded into fibers, fibers or the like.

# MELT PROCESSABLE THERMOTROPIC AROMATIC COPOLYESTERS AND PROCESS FOR PREPARING SAME

The present invention relates to a class of copolyesters which display optical anisotropy in the molten state and to articles such as fibers and films obtained from the copolyesters.

The present invention also relates to a process for preparing such copolyesters.

The present invention further relates to a process for preparing dihydroxy polyphenyls which can be used as staring material for the coplyesters.

Liquid crystalline polymers (LCPs) are macromolecules possessing significant orientation in either the molten state or in concentrated solution. The state of their solution (lyotropic) or melt (thermotropic) is between the boundaries of solid crystals and isotropic liquids. In the solid state these highly ordered polymers display exceptional strength properties in the direction of orientation. By designing molecules containing only relatively inert chemical bonds, preparation of thermally and oxidatively stable high-performance materials is possible.

A review of thermotropic LCPs can be found in Kwolek et al., "Liquid Crystalline Polymers", "Encyclopedia of Polymer Science and Engineering" 2nd Ed, Vol. 9, pp 23-55 (1987). Among those listed are polyesters. Many liquid crystalline polyesters display several of the desirable attributes of these compounds. Unfortunately, most have too high of a melt temperature (e.g. 400 °C or more) for economical melt fabrication.

There is a growing need in the thermoplastic engineering industries to provide for new and improved polyesters and copolyesters which possess a high degree of processability while concurrently exhibiting superior mechanical properties such as high tensile strength.

According to the invention, there is now provided a copolymer capable of forming an optically anisotropic melt comprising recurring structural units of Formula I, II, III and IV, and optionally V:

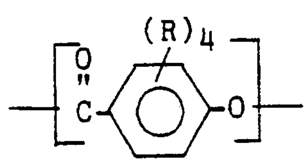 I,

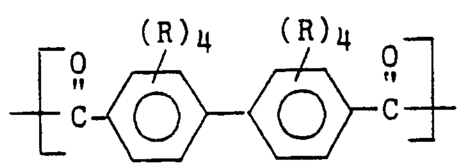 II,

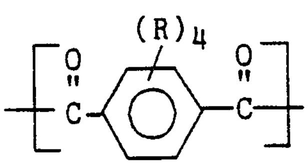 III,

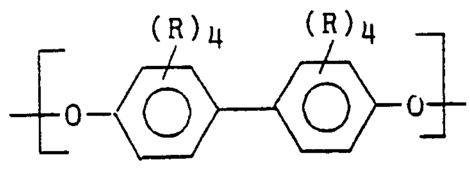 IV

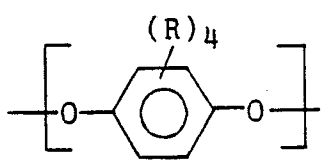 V,

wherein each R is independently a chemically inert substituent.

Another aspect of the present invention relates to a process for preparing the copolymers of Claim 1 which comprises subjecting a mixture of compounds of Formulas I′, II′, III′ and IV′, and optionally V′:

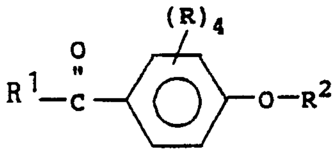 I',

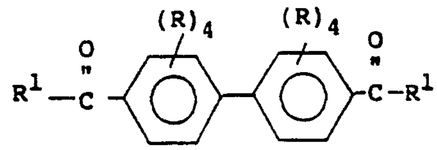 II',

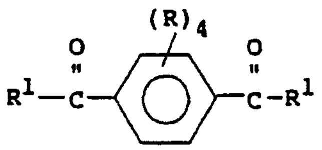 III',

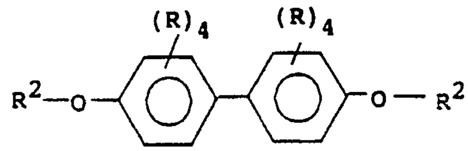 IV',

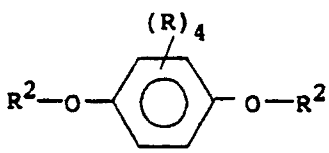 V',

wherein each R is independently a chemically inert substituent; each $R^1$ is independently hydroxyl, halogen or phenoxy; and each $R^2$ is independently hydrogen or acyl having 1 to 6, preferably 2 to 4 carbons to polymerization conditions to form the copolymer.

Still another aspect of the present invention relates to a process for the preparation of dihydroxy polyphenyls from the corresponding diisoalkyl polyphenyl compounds which can be monomers of the above-mentioned copolymer, the process comprising the steps of:

(a) contacting a diisoalkyl polyphenyl compound or an oxidation intermediate thereof with oxygen under reaction conditions sufficient to form a reaction mixture comprising a corresponding dihydroperoxyalkyl polyphenyl compound, and

(b) contacting the dihydroperoxyalkyl polyphenyl compound with a dissociating acid under reaction conditions sufficient to form a reaction product comprising the corresponding dihydroxy polyphenyl compound, characterized by separating the dihydroperoxyalkyl polyphenyl compound from the reaction mixture of step (a), and recycling the remaining reaction mixture as a starting material in step (a).

Preferably, R used in Formulas I through V and I′ through V′ is independently selected from the group consisting of hydrogen, halogen, lower alkyl such as $C_{1-3}$ alkyl, methoxy and phenyl. Most preferably, R is each occurrence hydrogen.

In the copolyesters of the invention the molar percent ranges for independently recurring units of Formulas II and III substantially equal the molar percent ranges of the independently recurring units of Formulas IV and V.

Preferred molar percent ranges for these copolyesters are from 20 mole percent to 60 mole percent of independently recurring units of Formula I, from 20 mole percent to 40 mole percent of independently recurring units of Formulas II and III wherein the ratio of Formula II units to Formula III units varies from 20:80 to 80:20, and from 20 mole percent to 40 mole percent of independently recurring units of Formulas IV and V wherein the ratio of Formula IV units to Formula V units varies from 20:80 to 100:0.

More preferred molar percent ranges are from 40 mole percent to 60 mole percent of independently recurring units of Formula I, from 20 mole percent to 30 mole percent of independently recurring units of Formulas II and III wherein the ratio of Formula II units to Formula III units varies from 25:75 to 75:25, and from 20 mole percent to 30 mole percent of independently recurring units of Formulas IV and V wherein the ratio of Formula IV units to Formula V units varies from 25:75 to 75:25.

The most preferred molar percent ranges are from 45 mole percent to 55 mole percent of independently recurring units of Formula I, from 22.5 mole percent to 27.5 mole percent of independently recurring units of Formulas II and III wherein the ratio of Formula II units to Formula III units varies from 33:67 to 67:33, and from 22.5 mole percent to 27.5 mole percent of independently recurring units of Formulas IV and

V wherein the ratio of Formula IV units to Formula V units varies from 33:67 to 67:33. The most preferred copolyesters of the invention melt below 350 ° C.

The copolymer of the present invention preferably have a weight average molecular weight of 2,000 to 200,000, more preferably 10,000 to 30,000. The molecular weight may be determined by standard techniques such as end group determination using infra red spectroscopy.

The copolymers may be formed by a variety of ester-forming techniques from difunctional organic compounds possessing functional groups which upon polycondensation form the requisite recurring units. For example, the functional groups of the organic aromatic compounds may independently contain carboxylic acid groups or acid halide groups and functional groups reactive therewith such as hydroxyl, or acyloxy groups. The hydroxy group is preferably esterified.

The differential organic compounds (monomers) which can be used in the present invention may be represented by the following formulas I′, II′, III′, IV′ and V′:

1 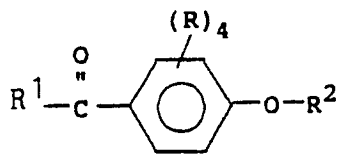 I',

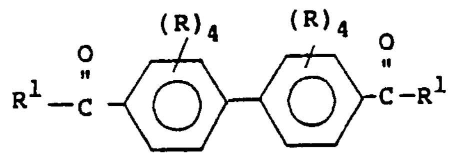 II',

2 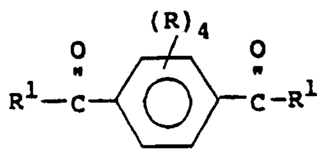 III',

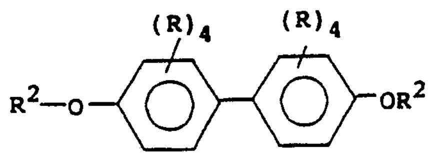 IV',

2 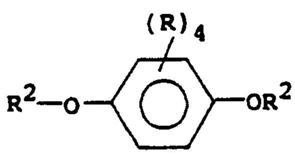 VI',

wherein R, $R^1$ and $R^2$ are as defined above. Preferably, $R^1$ is hydroxyl; hydrogen, and $R^2$ is hydrogen or acetyl. Thus, preferred reactants are (I′) 4-acetoxybenzoic acid; (II′) 4,4′-biphenyldicarboxylic acid; (III′) terephthalic acid; (IV) 4,4′-diacetoxybiphenyl; and (V′) 1,4-diacetoxybenzene.

The monomers can be polymerized by any of known techniques. For example, the organic compounds may be allowed to react under anhydrous conditions in an inert atmosphere via a melt acidolysis procedure, in a suitable solvent via a solution procedure, or in a heat exchange medium via a slurry polymerization as described in, U.S. Patent No. 4,067,852. Additional suitable reaction conditions are described in U.S. Patent No. 4,118,372. A preferable technique is the melt acidolysis technique.

Preferably, the polymerization can be carried out at a temperature of from 200 ° C to a temperature above the melting point, but below decomposition temperature of the resultant copolymer, more preferably from 260 to 360 ° C. The reaction time may range from 1 to 24 hours, preferably from 2 to 8 hours. The polymerization is preferably carried out at atmospheric pressure. It is preferable to reduce the pressure at the end of polymerization. Superatmospheric or subatmospheric pressures can also be used if desired.

A catalyst may or may not be used in the polymerization process. If one is used, representative catalysts for use in the process include dialkyl tin oxides (e.g., dibutyl tin oxide), diaryl tin oxides, titanium dioxide, alkoxy titanium silicates, titanium alkoxides, Lewis acids, hydrogen halides (e.g., HCl), alkali and alkaline earth metal salts of carboxylic acids (e.g., sodium acetate). The quantity of catalyst utilized typically is from 0.001 to 1 weight percent based upon total reactant weight, and most commonly from 0.01 to 0.2 weight percent. In a preferred method of polymerization, a catalyst is not used.

In another aspect, this invention is a process for the preparation of dihydroxy polyphenyl compounds which can be monomers for the copolymers of the present invention, the process comprising the steps of:

(a) contacting a diisoalkyl polyphenyl compound or an oxidation intermediate thereof with oxygen under reaction conditions sufficient to form a corresponding dihydroperoxyalkyl polyphenyl compound, and

(b) contacting the dihydroperoxyalkyl polyphenyl compound with a dissociating acid under reaction conditions sufficient to form the corresponding dihydroxy polyphenyl compound. characterized by separating the dihydroperoxalkyl polyphenyl compound from the reaction mixture of step (a), and recycling the remaining reaction mixture as a starting material in step (a).

The term "diisoalkyl polyphenyl compounds" as used herein refers to para-substituted biphenyl, terphenyl, or para-phenoxybiphenyl compounds having two isopropyl or isobutyl groups, or one of each, in the para positions, and are preferably compounds of the following formula:

$R^3$-$R^4$-$R^3$

wherein $R^4$ is biphenylene, terphenylene, or paraphenoxybiphenylene, and is preferably biphenylene, and $R^3$ is independently in each occurrence isopropyl or isobutyl, and the $R^3$ groups are substituted in the para,para′ positions of the $R^4$ groups. Examples of such compounds include 4,4′-diisopropylbiphenyl compounds, 4,4′-diisobutylbiphenyl compounds, and 4-isopropyl-4′-isobutylbiphenyl compounds. Diisoalkyl polyphenyl compounds for use in the process of this invention may be prepared by the alkylation of a polyphenyl using an acid catalyst. One example of such a method is described in U.S. application Serial No. 123,741 of G. J. Lee et al., "Alkylation of Polycyclic Aromatic Compounds to Alkylates Enriched in the Para-Substituted Isomers," filed November 23, 1987.

The term "oxidation intermediate" of diisoalkyl polyphenyl compounds as used herein refers to a derivative formed by the oxidation of a diisoalkyl polyphenyl compound which forms para,para′-dihydroperoxyalkylbiphenyl, para-hydroperoxyalkyl-para′-carbinolbiphenyl, or para,para′-dicarbinolbiphenyl upon further oxidation. Examples of such intermediates include 4-isopropyl-4′(2-hydroperoxy-2-propyl)-biphenyl and 4-carbinol-4′-(2-hydroperoxy-2-propyl)biphenyl.

The oxidation step may be carried out in any suitable reaction vessel into which an oxygen-containing gas may be introduced. This step is preferably carried out with the use of essentially pure oxygen or mixtures of oxygen with inert gases, but may also be carried out with the use of solids which release oxygen under process conditions. Air is generally used as the source of oxygen according to the process of this invention. The oxidation step may be carried out at any pressure above the vapor pressure of the reaction mixture, and the oxygen partial pressure is preferably above 34 kPa (5 psi), more preferably above 138 kPa (20 psi), and most preferably above 345 kPa (50 psi). Higher pressures increase the rate of oxidation, and upper limits on reaction pressures are advantageously determined by the pressure limitations of the process equipment. Preferably, the oxidation pressure is less than 3448 kPa (500 psig). The oxidation step may be carried out at any temperature which will permit or enable the oxidation of the polyphenyl compound, and is preferably above 60°C, more preferably above 80°C, and most preferably above 90°C, and is preferably below 150°C, more preferably below 120°C, and most preferably below 100°C. Temperatures above 80°C are preferred in order to keep the product soluble and achieve the highest conversion possible. Temperatures below 150°C are preferred to avoid decomposition of the dihydroperoxyalkyl products to ketones and carbinols.

The temperature at which the oxidation step is conducted will also affect the relative amount of dihydroperoxyalkyl polyphenyl which is formed. Lower temperatures of 80°C will promote the formation of a higher percentage of dihydroperoxyalkyl polyphenyls. Higher temperatures of 100°C will lead to the formation of a mixture containing a higher percentage of dicarbinol polyphenyl or 4-hydroperoxyalkyl-4′-carbinol polyphenyl. The duration of the oxidation step is determined by the rate of oxidation and the level of conversion desired. This oxidation step is preferably carried out by passing the oxygen-containing gas through the compound being oxidized under the above-described conditions of time, temperature, and oxygen pressure.

The oxidation step may be carried out neat, but is preferably carried out in the presence of an inert solvent. The use of a solvent facilitates the oxidation step since a partially-oxidized polyphenyl compound may be a solid at reaction conditions. Any organic solvent which is not miscible with aqueous basic solutions and is reasonably stable at oxidation reaction conditions is suitable. Benzene, ortho-dichlorobenzene, methylene chloride, $C_{8-20}$ paraffins, and toluene are preferred, and benzene is the most preferred solvent. When a solvent is employed, the molar ratio of diisoalkylated polyphenyl compound: solvent will advantageously depend on the polyphenyl compound's solubility in that particular solvent. Less solvent is needed when the diisoalkylated polyphenyl compound is extremely soluble in the particular solvent, although the solvent is preferably present in amounts sufficient to prevent the precipitation of any oxidation catalysts employed. Although polyphenyl compounds may exhibit a higher degree of solubility in other solvents, benzene is the most preferred solvent because it is easily separated from the reaction mixture. When benzene is used as a solvent, it is preferably present in a polyphenyl compound:benzene weight ratio of about 1:2.

The oxidation step is also preferably carried out in the presence of a suitable amount of a basic material

to neutralize acidic materials which can be formed as by-products in the oxidation reaction. Sodium hydroxide is the most preferred base in the process of the invention. When employed, the base is preferably present in a polyphenyl compound:base molar ratio in the range from 1 to 15, and more preferably 2 to 6. It is also preferable and within the scope of the invention to employ suitable oxidation initiators or catalysts, such as, for example, azo-bis-isobutyronitrile or $\alpha'$-azo- -bis(cyclohexane-1-carbonitrile), although the conversion of isoalkyl groups to hydroperoxide groups is self-initiating under most oxidation conditions. Other suitable oxidation catalysts are described in "Azo Catalysts", *Encyclopedia of Polymer Science*, 2(1), pp. 278-293 (1965). In one preferred embodiment, the initiator is para,para'-dihydroperoxyalkyl polyphenyl prepared by the oxidation step of the process of the invention.

Since the reaction is between heterogeneous phases, being between gaseous oxygen and liquid polyphenyl, suitable agitation is necessary. It is particularly important to bring the air, oxygen, or other oxygen-containing gas into intimate contact with the liquid phase. This may be effected, for example, by using high-speed stirrers, suitable nozzles, porous plates, or any combination thereof, together with sufficient oxygen pressure.

Although dihydroperoxyalkyl polyphenyls are the preferred product of the oxidation step, other dioxygenated materials may also be formed, including dicarbinol polyphenyl compounds and hydroperoxyalkyl carbinol polyphenyl compounds. The conversion of diisoalkyl polyphenyl compound to dioxygenated polyphenyl compound is preferably at least 10 percent, more preferably at least 20 percent, and most preferably at least 30 percent. The selectivity to the dioxygenated polyphenyl compound is preferably at least 80 percent, more preferably at least 90 percent, and most preferably at least 95 percent.

The dihydroperoxyalkyl polyphenyl or other dioxygenated polyphenyl materials may be separated from the reaction product, if so desired, by any suitable technique. In one embodiment, the crude oxidation mixture is mixed with an aromatic solvent to take up the polyphenyls, and is heated to form a two-phase mixture. Representative substituted or unsubstituted aromatic solvents which may be used include, for example, benzene, xylene, toluene, chlorobenzene, ortho-dichlorobenzene, anisole, or mixtures thereof, with ortho-dichlorobenzene as the most preferred solvent. The amount of aromatic solvent used in this extraction will generally be from 0.5 to 20, preferably from 1 to 2 parts by weight of aromatic solvent to one part by weight of reaction mixture. Temperatures used for this extraction will generally range from 10°C to 70°C, preferably from 20°C to 30°C.

The organic layer, after removal, is contacted with an aqueous base under reaction conditions sufficient to extract the dihydroperoxyalkyl polyphenyl and other dioxygenated materials away from the starting materials and mono-oxygenated materials into the base layer. This extraction step is preferably conducted at temperatures less than 90°C, and more preferably at ambient temperatures, since higher temperatures may facilitate the decomposition of the hydroperoxide groups of any polyphenyls containing such groups to carbinols. This step may be repeated several times and the base layers combined. The organic layer, containing unoxygenated material and mono-oxygenated material, is recycled as a starting material.

The aqueous base used in the extraction step can be water-soluble oxides, hydroxides, carbonates, bicarbonates, phosphates, or hydrogen phosphates of sodium, potassium, lithium, calcium, barium, strontium, or magnesium. The preferred aqueous basic material used in this extraction step is potassium hydroxide, preferably in a 10 to 30 weight percent aqueous solution, and more preferably in an about 15 weight percent aqueous solution. This relatively low concentration is preferred in order to minimize the decomposition of hydroperoxide groups present, especially at elevated temperatures. The amount of base used is preferably in a range from 0.5 moles to 4 moles, more preferably from 1 mole to 2 moles of base per mole of dihydroperoxyalkyl or dioxygenated polyphenyl compounds.

The base layers, after separation, and usually after combining, may be contacted with another solvent under conditions sufficient to extract the dihydroperoxyalkyl polyphenyl compound and other di-oxygenated materials into an organic layer. This step may also be repeated several times. The organic layers may then be concentrated to yield solid dihydroperoxyalkyl polyphenyl or other dioxygenated polyphenyl compounds. The solvent used in the extraction step may be any solvent which has some degree of water solubility and is not affected by concentrated acid or hydrogen peroxide, and is preferably acetone, methyl isobutyl ketone, methyl ethyl ketone, or methylene chloride, and most preferably is acetone. The solvent is used in a volume amount which is preferably equal to the amount of base solution. Preferably, the base solution is extracted two or three times and the organic layers are combined.

The second step of the process of the invention (hereafter referred to as the "acidolysis step") comprises contacting the dihydroperoxyalkyl-, dicarbinol-, or hydroperoxyalkyl carbinol-polyphenyl compound with a dissociating acid under reaction conditions sufficient to form the corresponding dihydroxy polyphenyl compound. This acidolysis step may be carried out in any suitable reaction vessel in accordance with conventional procedures for the acidolysis of hydroperoxides using acids as described, for

example, in U.S. Patents Nos. 2,626,281 and 2,628,983. In accordance with such procedures, the hydroperoxide group is decomposed at elevated temperatures, preferably above 50° C, in the presence of a dissociating acid.

Dissociating acids which may suitably be employed in the process of the invention are any acid with a pKa equal to or less than 5.0, and is preferably a strong mineral acid such as sulfuric acid, hydrochloric acid or strong organic acids such as acetic acid, trifluoroacetic acid, or paratoluene sulfonic acid. Most preferably, the acid employed is concentrated sulfuric acid. The acid is employed in a polyphenyl compound:acid molar ratio of above 1:3, and preferably below 1:0.6, and is most preferably 1:1.

The acidolysis step is preferably carried out in the presence of hydrogen peroxide in order to convert carbinol groups present to hydroperoxyalkyl groups, which may then be converted to hydroxy groups in the acidolysis step. The hydrogen peroxide is preferably present in a molar amount such that the hydrogen peroxide:carbinol functionality ratio is approximately 1:1.

The conversion of dihydroperoxyalkyl polyphenyl compound to dihydroxy polyphenyl compound is preferably at least 90 percent, and is most preferably at least 99 percent. The selectivity to the dihydroxy polyphenyl compound is preferably at least 90, and is most preferably at least 99 percent.

The dihydroxy polyphenyl compound may be separated from the reaction product by any suitable technique. In one preferred embodiment, the reaction product is neutralized with sodium bicarbonate to form an acid salt, and then filtered to remove the acid salt. These neutralization and filtration steps advantageously remove the acid catalyst from the reaction product while retaining the dihydroxy polyphenyl compound in solution in the reaction product.

In addition, the dihydroxy polyphenyl compound may be purified by any suitable technique. In one preferred embodiment, the dihydroxy polyphenyl compound is extracted from the filtered solution with potassium hydroxide, preferably in a 10 to 30 weight percent solution, and more preferably in an about 15 weight percent solution. The base layers are then combined and neutralized with an acid, preferably with a pKa of less than about 11, such as concentrated hydrochloric acid, and filtered to yield a dihydroxy polyphenyl product of typically greater than 99 percent purity, and preferably of greater than 99.9 percent purity.

As mentioned above with respect to the oxidation step, the unoxygenated and mono-oxygenated materials are preferably recycled as starting material after the dioxygenated materials are extracted using a basic material. When the unoxygenated material and mono-oxygenated material is recycled, the process of the invention is advantageously more efficient. When the oxidation step of the process is more selective to dihydroperoxyalkyl polyphenyls, less hydrogen peroxide is needed in the acidolysis step to convert dicarbinol polyphenyls to dihydroperoxyalkyl polyphenyls. However, oxidation reaction conditions which are more selective to dihydroperoxyalkyl polyphenyls give a lower conversion of oxidation starting material. Using this extra recycling step allows the oxidation process to be conducted economically under more selective conditions, since the unconverted starting material is not discarded as waste after separation. Further, the lower percentage of dicarbinol polyphenyls in the starting material for the acidolysis step means that less hydrogen peroxide is required in that step to convert all of the dicarbinol groups to hydroperoxide alkyl groups, which are then converted to hydroxy groups.

Biphenyl-4,4′-dicarboxylic acid, which is another monomer for the copolymer of this invention, can be prepared by any known techniques. For example, EPO 267,774 discloses a process for the preparation of biphenyl-4,4′-dicarboxylic acid, which comprise oxidizing 4,4′-diisopropyliphenyl or an oxidation intermediate thereof with molecular oxygen.

Liquid crystalline copolyester of this invention may be extruded into articles such as fibers which have outstanding strength and stiffness and will maintain their useful properties at elevated temperatures. Such fibers would be useful as tire cords, reinforcement in hoses, cables, conveyor belts or composite structures with matrixes prepared from other resinous materials. The films formed from the copolyesters will have excellent solvent and chemical resistance. In addition, the films will have low flammability and good electrical insulating properties. The films would be useful as cable wrap, electric motor dielectric film and wire insulation. In general, the copolyesters of the present invention are useful for the manufacture of shaped articles such as those which are injection molded possessing high strength, stiffness, chemical resistance and low flammability.

Conventional additives and processing aids can be added to the copolyester melts of the invention to improve the properties of articles made therefrom. Examples of additives are oxidation stabilizers; heat stabilizers; ultraviolet light (UV) stabilizers; lubricants; mold release agents; dyes and pigments; fibrous or powdered fillers and reinforcing agents; nucleating agents; and plasticizers.

Examples of oxidation stabilizers and heat stabilizers are halides of metals of group I of the Periodic Table, used alone and used as a mixture with copper (I) halides or sterically hindered phenols in

concentrations from 0.001 to 1 weight percent based on the weight of the copolyester composition.

Examples of UV stabilizers are substituted resorcinols, salicylates, benzotriazoles, benzophenones and mixtures of these. The stabilizers may be added, for example, in amounts from 0.001 to 2 weight percent based on the weight of the copolyester composition.

Dyes and pigments can be used, for example, in amounts from 0.001 to 5 weight percent based on the weight of the copolyester composition. Examples are nigrosine, titanium dioxide, cadmium sulfide, phthalocyanine dyes, ultramarine blue and carbon black.

Examples of fillers and reinforcing agents are carbon fibers, glass fibers, amorphous silica, calcium silicate, aluminum silicate, magnesium carbonate, kaolin, chalk, powdered quartz, mica and feldspar, which may be present in a concentration from 0.5 to 70 weight percent, based on the total weight of the filled material.

Examples of nucleating agents are talc, calcium fluoride, sodium phenylphosphonate, alumina and finely divided polytetrafluoroethylene. Suitably, the nucleating agent may be present in an amount from 0.001 to 1 percent by weight.

Plasticizers, such as phthalates, hydrocarbon oils and sulfonamides can be included in an amount of from 0.0001 to 20 weight percent, based on the weight of the composition.

Also included in the composition of the invention, in addition to or in partial replacement of the reactants of Formulas I, II, III, IV, or V are amounts of other aromatic polymerizable units whose presence do not interfere with the excellent mechanical properties of these copolyesters. Examples of such aromatic units comprising these additional repeating units are isophthalic acid, resorcinol, 4,4′-isopropylidenediphenol, 3,4′-biphenyldicarboxylic acid and 3-hydroxybenzoic acid.

According to the present invention, there is provided improved copolymers which possess a low melting point, preferably below 350° C while concurrently exhibiting superior mechanical properties such as high tensile strength.

The present invention will be further described with reference to the following examples.

Example I

Preparation of a Copolyester from 4-Acetoxybenzoic Acid, Terephthalic Acid, 1,4-Diacetoxybenzene, 4,4′-Biphenyldicarboxylic Acid and 4,4′-Diacetoxybiphenyl

The polymerization was run in a 1 L, single neck, round bottom flask fitted with a two neck adapter upon which were mounted a paddle stirrer and a 13 cm Vigreaux distillation column, distillation head, condenser and receiver. An amount of 4-acetoxybenzoic acid (228.5 g, 1.268 moles), terephthalic acid (51.89 g, 0.3123 mole), 4,4′-biphenyldicarboxylic acid (75.66 g, 0.3123 mole), 1,4-diacetoxybenzene (60.65 g, 0.3123 mole) and 4,4′-diacetoxybiphenyl (84.42 g, 0.3123 mole) were added to the reaction flask. The apparatus was evacuated and refilled with nitrogen. The flask was immersed in a molten salt bath preheated to 285° C. When the solid reactants had melted, stirring was started and the temperature was slowly increased to 350° C over a 100 minute period at atmospheric pressure. In the next 35 minutes the pressure was reduced to 2 mm Hg and maintained for 85 minutes. The vacuum was then released under nitrogen and the reaction vessel was removed from the 350° C salt bath. The reaction apparatus was disassembled and the flask was broken away from the cooled, tan polymer plug. The plug was sawed into chunks and then ground in a Wiley mill. The copolyester comprised recurring units of Formulas I, II, III, IV and V wherein R is hydrogen.

The inherent viscosity of the copolyester prepared as described above was computed from the equation, $n_{inh} = (1n\ n_{rel})/C$, where $1n\ n_{rel}$ is the natural logarithm of the relative viscosity and C is the concentration in grams (g) of copolymer per deciliter (dl) of solution. Relative viscosity is the ratio of the polymer solution flow time to solvent flow time in a capillary viscometer at 45° C. The solvent used was pentafluorophenol. The concentration was 0.1 gram copolymer per deciliter of solution. The copolyester had an inherent viscosity of 8.4 dl/g.

Melt temperature analysis was carried out using differential scanning calorimetry (DSC) on a 15 mg compressed pellet at a heating and cooling rate of 20° C per minute on a Mettler DSC-30 low temperature cell with a Mettler TC10A thermal analysis processor (Mettler Instrument Corp., Hightstown, NJ). The copolyester showed peak melting points at 326° C and 316° C on the first and second heating scans of the DSC. On cooling, the copolyester showed a crystallization exotherm at approximately 285° C.

The copolyester was solid-state heat treated for 12 hours at 292° C and at less than 0.1 mm Hg

pressure to advance its molecular weight. The apparatus used for this treatment was a 1-liter round bottom flask connected to a rotating evaporator which was connected to a nitrogen/vacuum manifold. The apparatus was purged with nitrogen and evacuated. The flask was then lowered into a preheated salt bath and rotated for 12 hours. The melt viscosity of the copolyester at 330° C and 352 seconds$^{-1}$ shear rate was increased from 211 poise to 1650 poise after solid-state heat treatment.

The solid-state advanced copolyester was dried under vacuum for 14 hours at 100° C and then injection molded into standard 1/8 inch (0.3 cm) tensile test bars using a BoyTM 30-M Injection Molding Machine (Boy Machines Inc., Exton, PA). The barrel temperature was held at 330° C, the mold temperature at 88° C and the injection pressure at 5 bars. Table I lists the tensile, flexural and impact properties of these bars measured using procedures described in ASTM Test No.'s D638, D790 and D256, respectively.

Optical anisotropy of the copolyester melts can be determined by examination of the materials with the use of an optical microscope. The equipment used for determining the optical anisotropy of the copolyesters of the present invention included a TH 600 hot stage, (Linkham Scientific Instruments LTD, Surrey, England) and a Nikon Optiphot Microscope equipped with crossed polarizers and a 35 mm camera (Nikon Instrument Group, Nikon, Inc., Garden City, NY). A thin film of the polymer was optically anisotropic above its DSC-determined melting temperature when observed through a polarizing microscope.

Table I

| PROPERTY | VALUE |
|---|---|
| Tensile Strength | 121,440, kPa (17,600 psi) |
| Tensile Modulus | 4,954,200 kPa (718,000 psi) |
| Elongation | 5.38 percent |
| Flexural Strength | 102,120 kPa (14,800 psi) |
| Flexural Modulus | 5,706,300 (827,000 psi) |
| Notched Izod Impact Strength | 489 J/m (9.14 ft lbs/inch) |

<u>Example II</u>

<u>Preparation of a Copolyester from 1,4-Diacetoxybenzene, Terephthalic Acid, 4,4′-Diacetoxybiphenyl, 4,4′-Biphenyldicarboxylic Acid and 4-Acetoxybenzoic Acid</u>

Terephthalic acid (51.89 g, 0.3123 mole), 4-acetoxybenzoic acid (228.5 g, 1.268 moles), 1,4-diacetoxybenzene (61.40 g, 0.3162 mole), 4,4′-biphenyldicarboxylic acid (75.66 g, 0.3123 mole), and 4,4′-diacetoxybiphenyl (84.42 g, 0.3123 mole) were added to a reaction flask and polymerized using the procedure of Example I. The copolyester comprised recurring units of Formulas I, II, III, IV and V wherein R is hydrogen.

The inherent viscosity as determined using the methods of Example I was 9.2 dl/g. The peak melt temperatures as determined using the methods of Example I were 328° C and 318° C on the first and second heating scans, respectively. The copolyester gave a crystallization exotherm at approximately 285° C on cooling.

The copolyester was injection molded using the methods of Example I. It was dried under vacuum. The mold temperature was 90° C, the barrel temperature was 335° C and the injection pressure was 800 kPa (eight bars). Table II shows the tensile, flexural and impact properties of the bars as determined by ASTM Test No.s D638, D790 and D256, respectively. The heat distortion temperature (HDT) was determined by thermomechanical analysis. A 9.9 mm in diameter pellet of the copolyester was compression molded and sanded to a 3.2 mm thick flat disk. A 9900 Thermal Mechanical Analyzer (DuPont Chemical Company) with a 0.635 mm diameter probe loaded with a 10 g weight was used for analysis of the sample. The temperature was increased at 5° C per minute under nitrogen. The onset of the depression of the sample

was taken as the HDT.

Table II

| PROPERTY | VALUE |
|---|---|
| Tensile Strength | 141,450 kPa (20,500 psi) |
| Tensile Modulus | 7,659,000 (1,110,000 psi) |
| Elongation | 3.55% |
| Flexural Strength | 117,300 kPa (17,000 psi) |
| Flexural Modulus | 9,453,000 kPa (1,370,000 psi) |
| Notched Izod Impact Strength | 186 J/M (3.47 ft lbs/inch) |
| HDT | 288° C |

A thin film of the polymer was optically anisotropic above its DSC-determined melting temperature when observed through a polarizing microscope.

Example III Copolyester of 4-Acetoxybenzoic Acid, Terephthalic acid, 4,4′-Biphenyldicarboxylic Acid, 4,4′-Diacetoxybiphenyl and 1,4-Diacetoxybenzene

An amount of 4-acetoxybenzoic acid (19.16 g, 0.1063 mole), terephthalic acid (4.35 g, 0.0262 mole), 4,4′-biphenyldicarboxylic acid (6.348 g, 0.0262 mole), 4,4′-diacetoxybiphenyl (9.437 g, 0.0349 mole) and 1,4-diacetoxybenzene (3.390 g, 0.0175 mole) were melt polymerized according to the methods of Example I to give an opaque, tan-colored copolyester. The copolyester comprised recurring units of Formulas I, II, III, IV and V wherein R is hydrogen. The DSC analysis as used in Example I, showed a broad melting endotherm from 295° C to 340° C on the first heating scan, a crystal lization exotherm at 290° C on cooling and a peak melting temperature of 320° C on the second heating scan.

A thin film of the polymer was optically anisotropic above its DSC-determined melting temperature when observed through a polarizing microscope.

Example IV Copolyester of 4-Acetoxybenzoic Acid, Terephthalic Acid, 4,4′-Biphenyldicarboxylic Acid and 4,4′-Diacetoxybiphenyl

An amount of 4-acetoxybenzoic acid (30.58 g, 0.1697 mole), terephthalic acid (6.944 g, 0.0418 mole), 4,4′-biphenyldicarboxylic acid (10.13 g, 0.0418 mole) and 4,4′-diacetoxybiphenyl (22.59 g, 0.0836 mole) were melt polymerized according to the methods of Example I to give an opaque, tan-colored copolyester. The copolyester comprised recurring units of Formulas I, II, III and IV wherein R is hydrogen. The DSC analysis as used in Example I, showed a peak melting temperature of 338° C on the first heating scan, a crystallization exotherm at 295° C on cooling and a broad melting endotherm from 300° C to 350° C on the second heating scan. A thin film of the polymer was optically anisotropic above its DSC-determined melting temperature when observed through a polarizing microscope using the methods of Example I.

Other copolyesters were prepared using the procedures of Examples I and II. The DSC-determined melting points (Tm) are shown in Table III. The table shows the mole fraction of each reactant wherein: 4-acetoxybenzoic acid (ABA), 4,4′-biphenyldicarboxylic acid (BPDCA), terephthalic acid (TA), 4,4′-diacetoxy biphenyl (DABP), 1,4-diacetoxybenzene (DAB) are the reactants.

Table III

| Mole Fraction of Each Reactant Added | | | | Melting Temp | |
|---|---|---|---|---|---|
| ABA | BPDCA | TA | DABP | DAB | Tm(°C) |
| 0.504 | 0.124 | 0.124 | 0.000 | 0.248 | 358 |
| 0.504 | 0.124 | 0.124 | 0.038 | 0.210 | 349 |
| 0.504 | 0.124 | 0.124 | 0.075 | 0.173 | 331 |
| 0.504 | 0.124 | 0.124 | 0.124 | 0.124 | 320 |
| 0.504 | 0.124 | 0.124 | 0.165 | 0.083 | 320 |
| 0.504 | 0.124 | 0.124 | 0.248 | 0.000 | 338 |

## Example V - Preparation of 4,4′-di(2-hydroperoxy-2-propyl)biphenyl

### Oxidation of 4,4′-diisopropylbiphenyl

A 100-g portion of 4,4′-diisopropylbiphenyl (DIPB) in 200 g of benzene, 200 g of 3 percent aqueous NaOH, and 1 g of azo-bis-isobutyronitrile (AIBN) were loaded in a 1-liter stirred titanium reactor with 175 kPa (25 psig) oxygen fed on demand at 80°C for 3 days. The conversion of DIPB is 94 percent, with 67 percent to 4,4′-di(2-hydroperoxy-2-propyl)biphenyl, 18 percent to 4-(2-hydroperoxy-2-propyl)-4′-carbinol biphenyl, and 5 percent to 4,4′-dicarbinolbiphenyl.

### Purification of 4,4′-di(2-hydroperoxy-2-propyl)biphenyl

The crude oxidation mixture was dissolved in 100 g of benzene and warmed to form a two-phase mixture. The organic layer containing the dioxygenated biphenyl products was separated and was extracted twice with 35 cc portions of a 15 weight percent aqueous potassium hydroxide solution. The aqueous base layers containing the dioxygenated biphenyl materials were combined and back extracted twice with 15 cc portions of acetone. The acetone layers containing the dioxygenated biphenyl materials were combined and concentrated to yield a white solid analyzed by liquid chromatography to be 4,4′-di(2-hydroperoxy-2-propyl )biphenyl.

## Example VI - Preparation of 4,4′-dihydroxybiphenyl

### Oxidation of 4,4′-Diisopropylbiphenyl

A 340 g portion of DIPB in 100 g of benzene, 200 g of 3 percent NaOH and 1 g of AIBN were loaded in a 1 l stirred titanium reactor with 350 kPa (50 psig) oxygen fed on demand at 95°C for 3 days. The conversion of DIPB is above 93 percent, with 3.7 percent to 4-carbinol-4′-isopropylbiphenyl, 14.1 percent to 4-(2-hydroperoxy-2-propyl)-4′-carbinolbiphenyl, and 72.9 percent to 4,4′-dicarbinolbiphenyl.

### Purification of 4,4′-Di(2-hydroperoxy-2-propyl)biphenyl

A portion of the reactor solids from Example 2 were dissolved in 100 cc of benzene and extracted three times with 65 cc of a 15 weight percent potassium hydroxide solution. Each of the base layers was back extracted twice with 60 cc of acetone. The acetone layers were combined and concentrated to give a solid product which was a mixture of the mono potassium salt of the dihydroperoxide, the hydroperoxycarbinol, and the dicarbinol. A yield of 76 percent across this step, based on the quantity of DIPB, was obtained.

Acidolysis of 4,4′-Di(2-hydroperoxy-2-propyl)biphenyl

A portion (5.0 g) of the crude oxidation solids of Example 2 containing 7.9 mmoles of difunctional equivalents were dissolved, in 65 cc of acetone and brought to reflux. To this solution was added 1.79 cc of a 30 weight percent aqueous hydrogen peroxide solution. 1.3 cc of concentrated sulfuric acid (18 N) was dissolved in 15 cc of cold acetone and added to the refluxing reaction mixture over 7 minutes. Reflux conditions were continued for 1 hr 25 min. The reaction product was cooled, excess sodium bicarbonate added and the mixture filtered. The acetone solution was extracted three times with 15 ml portions of 15 weight percent aqueous potassium hydroxide solution. The base layer was removed, acidified with concentrated hydrochloric acid and the resulting precipitated solids recovered by filtration, yielding 1.4 g of 4,4′-dihydroxy biphenyl as a tan solid for a 95 percent yield.

## Claims

1. A copolymer capable of forming an optically anisotropic melt comprising recurring structural units of Formula I, II, III, and IV:

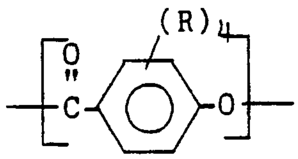 I,

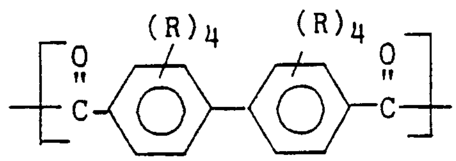 II,

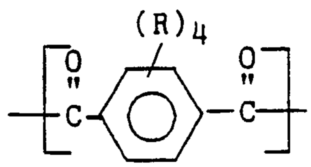 III,

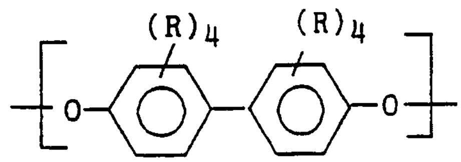 IV,

wherein each R is independently a chemically inert substituent.

2. The copolymer of Claim 1 which further comprises a recurring structural unit of Formula V:

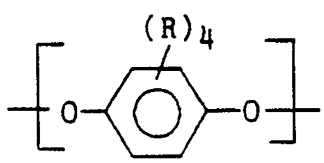 V

wherein each R is independently a chemically inert substituent.

3. The copolymer of Claim 1 wherein R is hydrogen, halogen, $C_{1-3}$ alkyl, methoxy or phenyl.

4. The copolymer of Claim 1 or 2 which comprises (a) 20 to 60 mole percent of the recurring unit of Formula I; (b) 4 to 32 mole percent of the recurring unit of formula II; (c) 4 to 32 mole percent of the recurring unit of Formula III; (d) 20 to 40 mole percent of the recurring unit of Formula IV; and (e) 0 to 32 mole percent of the recurring unit of Formula V.

5. An article made of the copolymer of Claim 1 or 2.

6. The article of Claim 5 which is a fiber or a film.

7. A process for preparing the copolymer of Claim 1 which comprises subjecting a mixture of compounds of Formulas I′, II′, III and IV:

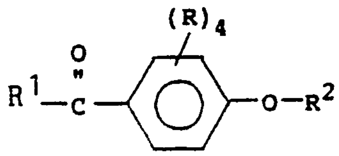 I',

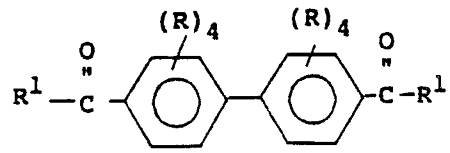 II',

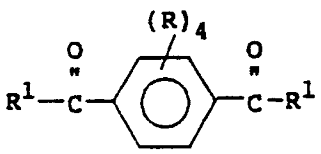 III',

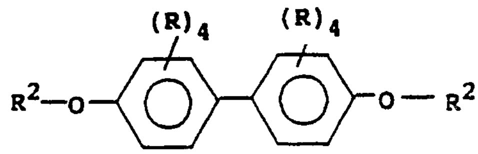 IV',

wherein each R is independently a chemically inert substituent; each $R^1$ is independently hydroxyl, halogen or phenoxy; and each $R^2$ is independently hydrogen or $C_{1-6}$ acyl, to polymerization conditions to form the copolymer.

8. The process of Claim 7 wherein said mixture further comprises a compound of Formula V':

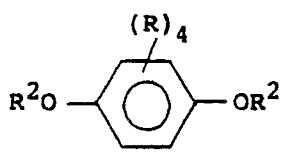 V',

wherein each R is independently a chemically inert substituent; each $R^2$ is independently hydrogen or $C_{1-6}$ acyl.

9. The process of Claim 7 wherein the polymerization is carried out at a temperature of from 200° C to a temperature below the decomposition temperature of the copolymer for 1 to 24 hours.

10. A process for the preparation of dihydroxy polyphenyls from the corresponding diisoalkyl polyphenyl compounds comprising the steps of:

(a) contacting a diisoalkyl polyphenyl compound or an oxidation intermediate thereof with oxygen under reaction conditions sufficient to form a reaction mixture comprising a corresponding dihydroperoxyalkyl polyphenyl compound, and

(b) contacting the dihydroperoxyalkyl polyphenyl compound with a dissociating acid under reaction conditions sufficient to form a reaction product comprising the corresponding dihydroxy polyphenyl compound,

characterized by separating the dihydroperoxyalkyl polyphenyl compound from the reaction mixture of step (a), and recycling the remaining reaction mixture as a starting material in step (a).